# EUROPÄISCHE PATENTSCHRIFT

(11) **EP 2 590 607 B1**
(45) Veröffentlichungstag und Bekanntmachung des Hinweises auf die Patenterteilung: **05.11.2014**
(21) Anmeldenummer: 11732375.8
(22) Anmeldetag: 05.07.2011
(51) Int. Cl.: A61F 13/15, A61F 13/56

(54) **ABSORBIERENDE INKONTINENZWEGWERFWINDEL MIT SEITENABSCHNITTEN**
ABSORBENT DISPOSABLE INCONTINENCE PANTS COMPRISING SIDE SECTIONS
COUCHE POUR INCONTINENCE ABSORBANTE ET JETABLE, COMPORTANT DES PARTIES LATÉRALES

(30) Priorität: 09.07.2010 DE 102010026643
(43) Veröffentlichungstag der Anmeldung: 15.05.2013
(73) Patentinhaber: Paul Hartmann AG, 89522 Heidenheim (DE)
(72) Erfinder: MALOWANIEC, Krzysztof-Daniel, 89522 Heidenheim (DE)
(86) Internationale Anmeldenummer: PCT/EP2011/003338
(87) Internationale Veröffentlichungsnummer: WO 2012/003965

(56) Entgegenhaltungen:
- EP-A1- 1 602 348
- WO-A2-2004/017882

## Beschreibung

Die Erfindung betrifft einen absorbierenden Einweg-Inkontinenzartikel des offenen Typs mithin eine Inkontinenzwegwerfwindel für Erwachsene mit einem Hauptteil, bestehend aus einem Vorderbereich, einem Rückenbereich und einem in Längsrichtung dazwischen liegenden zwischen den Beinen eines Benutzers zu liegen kommenden Schrittbereich, wobei der Hauptteil einen Saugkörper umfasst, und mit an den Rückenbereich und an den Vorderbereich beidseits angefügten, voneinander separaten mit Verschlussmitteln versehenen Seitenabschnitten, welche sich in Querrichtung über seitliche Längsränder des Hauptteils hinaus erstrecken und den Vorderbereich und den Rückenbereich im angelegten Zustand des Artikels miteinander verbinden.

Derartige Inkontinenzartikel sind bekannt und beispielsweise in WO 2005/102241 A1 beschrieben. Die Seitenabschnitte, die mitunter auch als Ohren bezeichnet werden, werden vorzugsweise im Cut & Place-Verfahren direkt, beidseitig an den Hauptteil, also das Chassis des Hygieneartikels angefügt. Diese Fertigungstechnologie erlaubt es, die Seitenabschnitte aus einem anderen Rohmaterial zu fertigen als den mittleren Hauptteil des Hygieneartikels. Beispielsweise können die Seitenabschnitte luftdurchlässig ausgeführt werden, wohingegen der mittlere Hauptteil im Wesentlichen feuchtigkeitsundurchlässig ausgebildet werden kann.

In US2008/0262461A1 ist ein Verfahren beschrieben, nach dem Seitenabschnitte mit elastischem Mittelteil an das Chassis einer Windel angefügt werden. Die Seitenabschnitte sind zur Beinöffnung hin bereichsweise kurvenförmig ausgebildet und weisen einen konvexen Abschnitt auf.

Die aus der Fertigungssicht effizienteste und einfachste sowie kostengünstigste Form der Seitenabschnitte ist die rechteckige Form. Sie erlaubt bei der Herstellung den Transport der die Seitenabschnitte bildenden Materialien in Form einer endlosen Flachmaterialbahn, von der dann die Seitenabschnitte quer zur Maschinenrichtung abgetrennt werden. Ein Schnittabfall ist hier praktisch nicht gegeben.

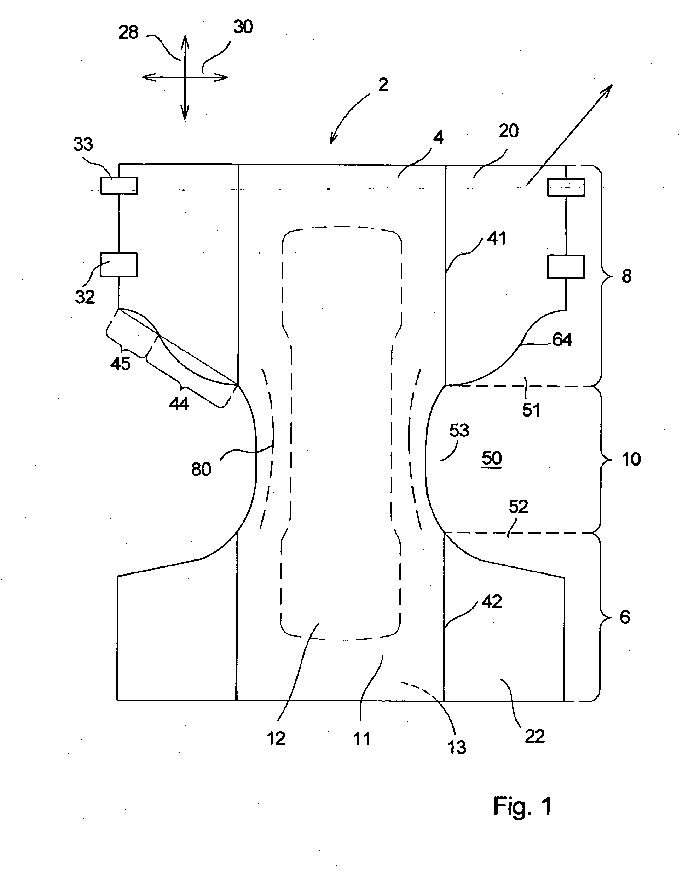

Es hat sich jedoch gezeigt, dass insbesondere bei der Ausbildung der Seitenabschnitte in der ansonsten vorteilhaften Rechteckform beim Anlegen und Tragen des Hygieneartikels mitunter das Problem besteht, dass die angefügten Seitenabschnitte im Bereich der seitlichen Längsränder des Hauptteils einreißen können. Es hat sich nämlich gezeigt, dass Anwender beim Anlegen des Hygieneartikels geneigt sind, einen zur Quer- und Längsrichtung des Hygieneartikels schrägen Zug auf die im Falle von Inkontinenzartikeln für Erwachsene extrem weit in Quer- und Längsrichtung ausladenden Seitenabschnitte auszuüben, was in Figur 1 mit einem schräg nach oben geneigten Pfeil angedeutet ist. Es kann solchenfalls vorkommen, dass Seitenabschnitte entlang der seitlichen Längsränder des Hauptteils einreißen, wobei der Riss ausgehend von dem dem Schrittbereich zugewandten Querrand des Seitenabschnitts ausgeht. Bislang wurde versucht, die Anfügung von derartigen Seitenabschnitten an den Hauptteil von Hygieneartikeln durch ein optimiertes Fügemuster zu verbessern, gemäß WO 2004/017882 A2 und WO 02/17843 A2.

Ein weiterer aus dem Stand der Technik bekannter Vorschlag ist, die Seitenabschnitte mit einem Verstärkungsmittel zu versehen, welches in Querrichtung betrachtet schmäler ausgebildet ist als ein jeweiliger Seitenabschnitt und welches wenigstens in einem den seitlichen Längsrand des Hauptteils überbrückenden Bereich vorgesehen ist, also sowohl einen seitlichen Längsrandbereich des Hauptteil als auch einen Teil des Seitenabschnitts in Querrichtung überfängt (DE102006050971A1).

Der vorliegenden Erfindung liegt die Aufgabe zugrunde, das vorstehend geschilderte Problem noch effektiver zu lösen, also absorbierende Inkontinenzwegwerfwindeln mit zwei hinten und vorzugsweise auch vorn angestückten und angefügten Seitenabschnitten zu schaffen, bei denen das Ausreißverhalten der hinteren Seitenabschnitte signifikant verbessert ist.

Diese Aufgabe wird bei einer absorbierenden Inkontinenzwegwerfwindel der eingangs genannten Art erfindungsgemäß dadurch gelöst, dass ein unterer Rand der hinteren Seitenabschnitte kurvenförmig ausgebildet ist, dass der untere Rand einen konvexen Abschnitt aufweist, dass das Verhältnis der Länge A des unteren Randes eines hinteren Seitenabschnittes zur der Breite B des unteren Randes eines hinteren Seitenabschnittes 0,4-0,9 beträgt und das Verhältnis der Längserstreckung E eines Extrempunktes P des konvexen Abschnittes zu dessen Quererstreckung F 0,15-0,80 beträgt, wobei A, B, E und F wie weiter unten anhand der Figurenbeschreibung beschrieben ermittelt werden.

Wie ebenfalls anhand der Figurenbeschreibung noch weiter erläutert werden wird, wird im Rahmen der vorliegenden Erfindung unter dem Extrempunkt P ein Punkt auf einem konvexen Abschnitt des unteren Randes der hinteren Seitenabschnitte verstanden, welcher einen maximalen Abstand von einer gedachten Geraden aufweist, welche gezogen ist durch die Endpunkte des unteren Randes eines hinteren Seitenabschnitts. Diese Endpunkte sind die Punkte, in denen der untere Rand den inneren bzw. den äußeren Rand der Seitenabschnitte trifft.

Überraschenderweise führt diese erfindungsgemäße Geometrie der hinteren Seitenabschnitte zu einem verbesserten Dehnungsverhalten der Seitenabschnitte in Gebrauch und damit letztlich zu deutlich verbessertem Ausreißverhalten der hinteren Seitenabschnitte, ohne dass die Passform der Inkontinenzwegwerfwindel beeinträchtigt ist.

Der konvexe Abschnitt des unteren Randes eines hinteren Seitenabschnittes, die Lage des Extrempunktes, also gleichsam die Koordinaten des Maximums des konvexen Abschnitts in Kombination mit dem Verhältnis der Längs- und Quererstreckung des unteren Randes eines hinteren Seitenabschnittes ermöglichen ein verbessertes Ausreißverhalten der hinteren Seitenabschnitte. Erklären lässt sich dies durch eine vergleichsweise hohe Arbeit (in mNm), welche in einem den Gebrauch simulierenden Seitenabschnittsausreißversuch bis zum Bruch der Materialien erforderlich ist. In Gebrauch bedarf es also mehr Energie, die Zerstörung des Materials herbeizuführen.

Vorzugsweise beträgt die Arbeit, welche in einem den Gebrauch simulierenden, unten näher beschriebenen Seitenabschnittsausreißversuch bis zum Bruch der Materialien erforderlich ist, zumindest 1900 mNm, insbesondere mindestens 1950 mNm, weiter insbesondere mindestens 2000 mNm, vorzugsweise jedoch weniger als 4000 mNm. Bevorzugt beträgt das Verhältnis der Länge A des unteren Randes eines hinteren Seitenabschnittes zu der Breite B des unteren Randes eines hinteren Seitenabschnittes 0,50-0,80, insbesondere 0,55-0,75.

In Weiterbildung der Erfindung wird vorgeschlagen, dass das Verhältnis der Quererstreckung F des Extrempunktes P zu der Quererstreckung B eines jeweiligen unteren Randes der hinteren Seitenabschnittes 0,20-0,60, insbesondere 0,30-0,50 beträgt.

Weiter bevorzugt beträgt das Verhältnis der Längserstreckung E des Extrempunktes P zur der Länge A eines unteren Randes eines jeweiligen hinteren Seitenabschnitts 0,10-0,40, insbesondere 0,10-0,30.

Vorteilhaft beträgt das Verhältnis der Längserstreckung E eines Extrempunktes P des konvexen Abschnittes zu dessen Quererstreckung F 0, 20-0,60 insbesondere 0,20-0,50.

Vorzugsweise beträgt die Weiterreißfestigkeit Fm des die hinteren Seitenabschnitte bildenden Materials gemessen und ermittelt als mittlere Kraft Fm wie unten näher beschrieben in Windellängsrichtung mindestens 4,0 N. Dies ermöglicht bei erfindungsgemäßer Konturierung der Seitenabschnitte, die Verschlussmittel sehr dicht, das heißt vorzugsweise höchstens 8,0 cm davon beabstandet an den unteren Rand der hinteren Seitenabschnitte zu positionieren. Es wird zwar nicht verkannt, dass die oben beschriebene Gefahr des Einreißens der Seitenabschnitte entlang der seitlichen Längsränder des Hauptteils mit der Entfernung der Verschlussmittel von dem unteren Rand der Seitenabschnitte abnimmt; gleichzeitig ginge damit jedoch ein erheblicher Verlust an Komfort einher, die Windel mit den sehr weit in Windellängs- und - querrichtung ausladenden Seitenabschnitten passgenau an einer Person anzulegen. Das passgenaue Anlegen der Windel lässt sich mit sehr dicht an dem unteren Rand der Seitenabschnitte positionierten Verschlussmitteln wesentlich einfacher bewerkstelligen, da hierdurch über die Verschlussmittel ein Zug auf nahezu die gesamte Seitenabschnittslänge ausgeübt werden kann.

Zur Herstellung einer erfindungsgemäßen Inkontinenzwegwerfwindel werden vorzugsweise zunächst rechteckförmige Materialabschnitte beidseits an den Windelhauptteil angefügt und die Seitenabschnittsbeinöffnungsbereiche erst anschließend durch einen kontinuierlich oder quasikontinuierlich geführten Abtrennvorgang, insbesondere durch einen Schnitt oder eine Stanzung, gebildet, so dass ein stetiger durchgehender Rand gebildet wird. Die DE102009015041 zeigt dieses Verfahren am Beispiel einer Inkontinenzwegwerfwindel mit konkav konturierten Seitenabschnitten. Die Linie des Abtrennvorgangs erfasst dabei den hinteren Seitenabschnitt, den Hauptteil und den vorderen Seitenabschnitt. Die Beinöffnungsbereiche sind somit vorzugsweise ausschließlich durch Schnitt- oder Trennkanten des einzigen, kontinuierlichen oder quasikontinuierlichen Abtrennvorgangs gebildet, was natürlich auch eine wirtschaftliche Herstellbarkeit der Inkontinenzwegwerfwindel impliziert und unerwünschte Kanten vermieden werden.

Bei diesem Abtrennvorgang muss in jedem Fall der von dem hinterem Seitenabschnitt, Hauptteil und vorderem Seitenabschnitt gebildete zusammenhängende Schnittabfall aus dem Prozess abgeführt werden. Dies erfolgt vorteilhaft nach dem in DE102008056220 beschriebenen Verfahren. Diesbezüglich wird vollumfänglich und ausdrücklich auf den Offenbarungsgehalt der DE102008056220 Bezug genommen.

Denkbar wäre auch, die Konturierung der Seitenabschnitte vor dem Anfügen der Seitenabschnitte an den Hauptteil vorzunehmen, beispielsweise an einer noch endlosen Seitenabschnittsbahn.

Nach einem weiteren Erfindungsgedanken beträgt ein Abstand C der Verschlussmittel, insbesondere der beinöffnungsnahen Verschlussmittel, von dem unteren, dem Schritt zugewandten Rand der hinteren Seitenabschnitte höchstens 8,0 cm, vorzugsweise höchstens 7, 0, besonders bevorzugt höchstens 6,5 cm und ganz besonders mindestens 0,5 cm und weiter insbesondere mindestens 1,0 cm.

Das Flächengewicht des die hinteren Seitenabschnitte bildenden Materials sollte vorzugsweise 14-40 g/m², insbesondere 16-30 g/m² und ganz besonders 17-28 g/m² betragen.

Die Weiterreißfestigkeit des die hinteren Seitenabschnitte bildenden Materials gemessen und ermittelt als mittlere Kraft Fm wie unten näher beschrieben beträgt vorzugsweise mindestens 5,0 N, besonders bevorzugt mindestens 6,0 N und ganz besonders bevorzugt mindestens 6,5 N, vorzugsweise jedoch höchstens 10,0 N.

Die Weiterreißfestigkeit des die hinteren Seitenabschnitte bildenden Materials gemessen und ermittelt als Mittelwert der Kraftspitzen Fm.sp wie unten näher beschrieben beträgt vorzugsweise mindestens 5,5 N, besonders bevorzugt mindestens 6,0 N, ganz besonders bevorzugt mindestens 6,5 N und insbesondere mindestens 7,0 N, vorzugsweise jedoch höchstens 12,0 N.

Die Weiterreißfestigkeit des die hinteren Seitenabschnitte bildenden Materials gemessen und ermittelt als maximale Spitzenkraft Fsp wie unten näher beschrieben beträgt vorzugsweise mindestens 5,5 N, besonders bevorzugt mindestens 6,0 N, ganz besonders bevorzugt mindestens 6,5 N und insbesondere mindestens 7,0 N, vorzugsweise jedoch höchstens 12 N.

In weiterer Weiterbildung der Erfindung hat es sich als vorteilhaft erwiesen, auch die vorderen Seitenabschnitte mit einem Flächengewicht wie oben für die hinteren Seitenabschnitte beschrieben zu versehen. Vorzugsweise weisen außerdem auch die vorderen Seitenabschnitte eine Weiterreißfestigkeit Fm und/oder Fm.sp und/oder Fsp wie für die hinteren Seitenabschnitte beschrieben auf.

Die vorderen Seitenabschnitte können an sich jede beliebige Form aufweisen, insbesondere rechteckförmig sein. Bevorzugt weisen die vorderen Seitenabschnitte jedoch ebenfalls eine Konturierung auf, insbesondere kann ein schrittzugewandter Querrand der vorderen Seitenabschnitte einen konkaven und/oder einen konvexen Abschnitt aufweisen.

Nach einer bevorzugten Ausführungsform der Erfindung verlaufen ein innerer Rand und ein äußerer Rand der vorderen und/oder hinteren Seitenabschnitte parallel zueinander. Weiter bevorzugt verlaufen der innere und/oder äußere Rand zumindest abschnittsweise, vorzugsweise gänzlich, also über ihre gesamte Erstreckung parallel zu einer Längsrichtung der Inkontinenzwegwerfwindel. Vorzugsweise weist der innere Rand eine größere Erstreckung D in der Längsrichtung auf als der äußere Rand.

Es hat sich weiter als vorteilhaft erwiesen, dass die vorderen und/oder hinteren Seitenabschnitte aus einem Vliesstoffmaterial bestehen oder ein Vliesmaterial umfassen. Geeignet sind insbesondere alle Vliesstoffmaterialien, die zumindest eine Rezepturkomponente auf Basis eines thermoplastischen Polymers enthalten. Die Vliesstoffe können Fasern aus PE, PP, PET, Rayon, Cellulose, PA und Mischungen dieser Fasern enthalten. Auch Bi- oder Multikomponentenfasern sind denkbar und vorteilhaft. Vorteilhaft sind insbesondere Kardenvliese, wasserstrahlvernadelte Vliese, Spunbond(S)-Vliese, Meltblown(M)-Vliese, SMS-Vliese, SMMS-Vliese oder auch Laminate aus einer oder mehreren dieser Vliesarten, wobei S für Spunbond- und M für Meltblown-Vliesschichten steht. Denkbar und vorteilhaft ist des Weiteren, die vorderen und/oder hinteren Seitenabschnitte aus einem Vliesstoff-Folienlaminat zu bilden. Solchenfalls würde die Folienkomponente außen zu liegen kommen und die Vliesstoffkomponente innen, um körperzugewandt eine weiche Oberfläche zu gewährleisten. In Weiterbildung dieses Erfindungsgedankens ist vorteilhaft, die vorderen und/oder hinteren Seitenabschnitte aus einem Vliesstoff-Folie-Vliesstofflaminat zu bilden, bei dem eine Folienkomponente sandwichartig zwischen zwei Vlieskomponenten angeordnet ist.

Des Weiteren erweist es sich als vorteilhaft, dass seitlich neben den Längsrändern des Saugkörpers erste elastische Elemente mit einer Komponente in Längsrichtung an den Hauptteil angefügt sind. Diese elastischen Elemente können exakt, das heißt geradlinig in Längsrichtung verlaufen oder besonders vorteilhaft auch einer gewissen Konturierung entlang der Beinöffnungen folgend vorgesehen werden. Die elastischen Elemente nehmen solchenfalls einen gekrümmten Verlauf entlang der Beinöffnung. In besonderer Weiterbildung dieses Erfindungsgedankens ist vorgesehen, dass die elastischen Elemente sich nicht in die Seitenabschnitte hinein erstrecken, sondern auf eine Positionierung innerhalb des Hauptteils limitiert sind. Des Weiteren können in der ersten Längsrichtung erstreckte zweite elastische Elemente, insbesondere in Form von so genannten und an sich zum Beispiel auch aus EP0263720A1 bekannten aufstehenden Cuff-Elementen, an die Windelhauptteilbahn angefügt werden. Diese vorzugsweise aufstehenden zweiten elastischen Elemente flankieren gewissermaßen ein Zentrum des Windelhauptteils oder Saugkörpers; sie können im Bereich der Saugkörperränder, innerhalb der Saugkörperränder oder außerhalb der Saugkörperränder vorgesehen werden. Sie bilden einen Seitenauslaufschutz der Inkontinenzwegwerfwindel.

In Weiterbildung der Erfindung ist vorgesehen, dass die Verschlussmittel zum bestimmungsgemäßen Festlegen der Inkontinenzwegwerfwindel an den Körper eines Menschen zumindest bereichsweise sowohl an der Außenseite des Hauptteils als auch an der Außenseite der vorderen Seitenabschnitte lösbar festlegbar sind, wobei die Haltekräfte zwischen den Verschlussmitteln und der Außenseite der vorderen Seitenabschnitte vorzugsweise größer sind als die Haltekräfte zwischen den Verschlussmitteln und der Außenseite des Hauptteils. Dies veranlasst den Benutzer in der Mehrzahl der Fälle, die Verschlussmittel an den vorderen Seitenabschnitten festzulegen. Die Haltekräfte als ÜberBauch-Haltekräfte ermittelt zwischen den insbesondere mechanischen Verschlusshilfen aufweisenden Verschlussmitteln und der Außenseite des Hauptteils betragen vorzugsweise 20-57 N/25mm, insbesondere 25-50 N/25mm. Des Weiteren betragen die Über-Bauch-Haltekräfte zwischen den Verschlussmitteln und der Außenseite der Seitenabschnitte im Vorderbereich vorzugsweise 58-90 N/mm, insbesondere 60-80 N/25mm. Des Weiteren erweist es sich als vorteilhaft, wenn die Über-Bauch-Haltekräfte zwischen den Verschlussmitteln und der Außenseite der hinteren Seitenabschnitte geringer sind als die Über-Bauch-Haltekräfte zwischen den Verschlussmitteln und der Außenseite der vorderen Seitenabschnitte. Auch dies veranlasst den Benutzer in der Mehrzahl der Fälle, die Verschlussmittel an den vorderen Seitenabschnitten festzulegen. Im Rahmen der vorliegenden Erfindung wurden die Über-Bauch-Haltekräfte nach der in WO2008049546A1 beschriebenen Prüfmethode ermittelt.

Vorteilhafterweise weisen die hinteren Seitenabschnitte mindestens ein weiteres beinöffnungsfernes mit Verschlusshilfen versehenes Verschlussmittel auf.

Die Außenseite des Hauptteils der Inkontinenzwegwerfwindel wird vorzugsweise zumindest bereichsweise, insbesondere aber vollflächig durch einen Vliesstoff gebildet. Dies vermittelt der Inkontinenzwegwerfwindel einen "textile-like" Eindruck. Solchenfalls ist es vorteilhaft, das Backsheet des Hauptteils aus einem Vliesfolienlaminat zu bilden, wobei die Vlieslage außen und die Folienlage innen zum Saugkörper gerichtet zu liegen kommt, so dass die Vlieslage die Außenseite des Hauptteils bildet. Damit ist zum einen die Flüssigkeitsundurchlässigkeit des Hauptteils sichergestellt und zum anderen der hautfreundliche Charakter der Windel gesichert. Die Folienlage dieses Vliesfolienlaminates ist dann vorzugsweise aus einer ein- oder mehrschichtigen flüssigkeitsundurchlässigen, vorzugsweise aber gleichwohl atmungsaktiven Folie gebildet, wobei die Atmungsaktivität der vorderen und/oder der hinteren Seitenabschnitte vorzugsweise größer ist als die Atmungsaktivität des das Backsheet der Inkontinenzwegwerfwindel bildenden Vliesfolienlaminates.

Vorteilhaft unterscheiden sich die hinteren Seitenabschnitte von den vorderen Seitenabschnitten bezüglich mindestens einer, insbesondere mindestens zweier, weiter insbesondere mindestens dreier und weiter insbesondere mindestens vierer ihrer Primäreigenschaften ausgewählt aus der Gruppe Art des Materials, Flächengewicht, Atmungsaktivität, Dichte, Dehnbarkeit, Verschlusskraft, Flächenerstreckung, Dicke, Farbe. Diesbezüglich wird hiermit ausdrücklich auf den Offenbarungsgehalt der WO2009/015746 Bezug genommen.

In Weiterbildung der Erfindung erweist es sich als vorteilhaft, wenn die Länge der vorderen und/oder hinteren Seitenabschnitte, also deren maximale Erstreckung in Windellängsrichtung mindestens 15 cm, insbesondere mindestens 20 cm, weiter insbesondere mindestens 25 cm, weiter insbesondere mindestens 27 cm und weiter insbesondere höchstens 45 cm beträgt. Vorteilhafterweise beträgt die Gesamtlänge der Inkontinenzwegwerfwindel 60-120 cm, insbesondere 65-115 cm und weiter insbesondere 70-110 cm. In Weiterbildung der Erfindung erweist es sich als vorteilhaft, wenn die Breite der vorderen und/oder hinteren Seitenabschnitte, also die maximale Erstreckung der Seitenabschnitte über den Seitenrand des Windelhauptteils hinaus 10-40 cm, insbesondere 12-35 cm, weiter insbesondere 13-30 cm beträgt. Vorzugsweise weisen die vorderen Seitenabschnitte die gleiche Breite auf wie die hinteren Seitenabschnitte.

In der Zeichnung zeigen
Figur 1 eine nicht maßstabsgerechte Draufsicht auf eine Inkontinenzwegwerfwindel in schematischer Darstellung mit beidseits angefügten Seitenabschnitten;
Figuren 2 eine vergrößerte Teilansicht der Inkontinenzwegwerfwindel nach Figur 1;
Figur 3 beispielhaft eine weitere Seitenabschnittsgeometrie einer erfindungsgemäßen Inkontinenzwegwerfwindel (nicht maßstabsgerecht)
Figuren 4 und 5 eine Darstellung der Prüfung der Seitenabschnittsausreißfestigkeit.

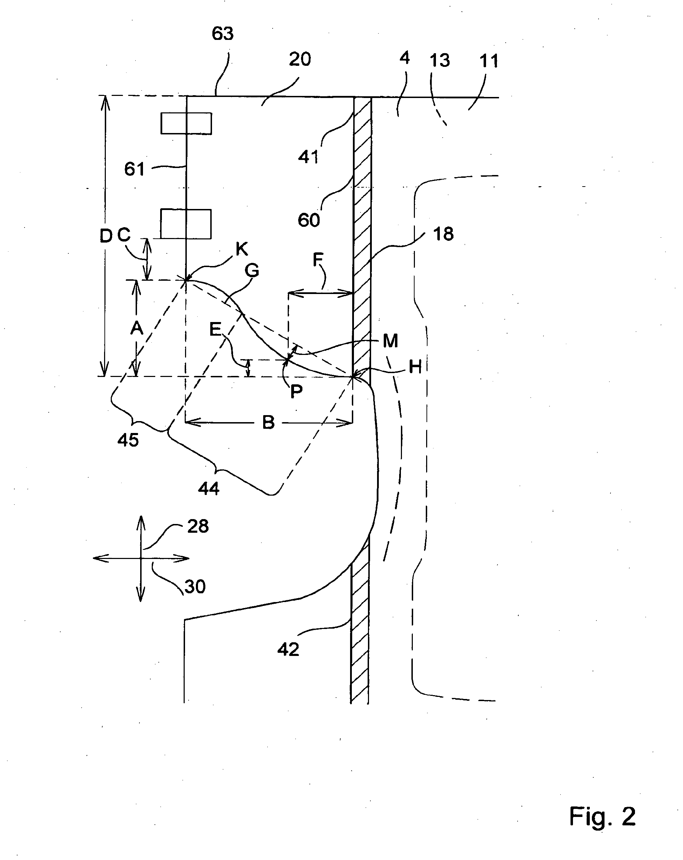

Figur 1 zeigt schematisch, nicht maßstabsgerecht eine Draufsicht auf die Innenseite, also die körperzugewandte Seite einer ersten Ausführungsform (Beispiel 1) der absorbierenden Inkontinenzwegwerfwindel 2 des offenen Typs in eben ausgefaltetem Zustand. Die Inkontinenzwegwerfwindel 2 umfasst einen Hauptteil 4 mit einem Vorderbereich 6, einem Rückenbereich 8 und einem in Längsrichtung 28 dazwischen liegenden Schrittbereich 10. Außerdem angedeutet ist ein Saugkörper 12, der üblicherweise zwischen chassisbildenden Materialien des Hauptteils 4, also insbesondere zwischen einem flüssigkeitsdurchlässigen aus einem Vliesstoffmaterial gebildeten Topsheet 11 und einem im Wesentlichen flüssigkeitsundurchlässigen aus einem Folienmaterial gebildeten Backsheet 13 des Hauptteils 4 angeordnet ist. Das Backsheet 13 kann auch aus einem flüssigkeitsundurchlässigem Vliesstoff oder einem Vliesfolienlaminat gebildet sein, wobei die Vlieslage dann außen und die Folienlage innen zum Saugkörper gerichtet zu liegen kommt. Dies vermittelt der Inkontinenzwegwerfwindel 2 einen "textile-like" Eindruck. Seitlich neben den Längsrändern des Saugkörpers 12 sind erste elastische Elemente 80 an den Hauptteil 4, zwischen Topsheet 11 und Backsheet 13 angefügt. Die elastischen Elemente 80 verlaufen im Wesentlichen in der Längsrichtung 28, also mit einer wesentlichen Komponente in Längsrichtung, wobei sie im dargestellten Fall einen gekrümmten Verlauf entlang des dem Schrittbereich 10 zuzuordnenden Beinöffnungsbereichsabschnittes nehmen. Die Inkontinenzwegwerfwindel 2 umfasst des Weiteren vordere Seitenabschnitte 22 und hintere Seitenabschnitte 20, die als vier separate Vliesstoffkomponenten beidseits an den Hauptteil 4 angefügt sind. Der untere Rand 64 der hinteren Seitenabschnitte 20 weist einen konvexen Abschnitt 44 und einen konkaven Abschnitt 45 auf, während die vorderen Seitenabschnitte 22 eine durchgehend konkave Beinöffnungskontur aufweisen. Wie eine vergrößerte nicht maßstabsgerechte Darstellung einer Teilansicht der Figur 1 zeigt (Figur 2) sind die Seitenabschnitte 20, 22 in einem schraffiert dargestellten Überlappungsbereich 18 mit chassisbildenden Materialien des Hauptteils 4, also beispielsweise mit dem Backsheet 13 und/oder dem Topsheet 11 unlösbar verbunden. Die Seitenabschnitte 20, 22 erstrecken sich über die vorderen und hinteren seitlichen Längsränder 42, 41 des Hauptteils in Querrichtung 30 hinaus.

Unter vorderen und hinteren seitlichen Längsrändern 42, 41 des Hauptteils werden im Rahmen der vorliegenden Erfindung diejenigen Längsrandbereiche des Hauptteils verstanden, an die die Seitenabschnitte angefügt sind und über welche diese sich hinaus erstrecken.

Die Längserstreckung der vorderen und hinteren Seitenränder des Hauptteils 42, 41 definieren damit auch die Längserstreckung des Vorderbereiches 6 und des Rückenbereiches 8 der Inkontinenzwegwerfwindel 2.

Es wird hiermit außerdem klargestellt, dass die Begriffe "Seitenrand" und "seitlicher Längsrand" vor- und nachstehend synonym verwendet werden.

Die Seitenabschnitte 20, 22 sind dafür gedacht und bestimmt, im angelegten Zustand der Inkontinenzwegwerfwindel 2 miteinander verbunden zu werden, um einen in Umfangsrichtung durchgehenden Hüftbereich des Hygieneartikels zu bilden. Dabei werden jeweils die auf einer Seite des Hauptteils 4 vorgesehenen Seitenabschnitte 20, 22 miteinander verbunden. Hierzu sind an den hinteren Seitenabschnitten 20 beinöffnungsnahe mechanische Verschlussmittel 32 und beinöffnungsferne Verschlussmittel 33, insbesondere mit mechanischen Verschlusshilfen wie Kletthaken, vorgesehen, welche auf der Außenseite der vorderen und hinteren Seitenabschnitte 20, 22 lösbar festlegbar sind. Vorzugsweise sind die Verschlussmittel außerdem auf der Außenseite des Hauptteils lösbar festlegbar, so dass der Inkontinenzartikel sehr variabel an die anatomischen Gegebenheiten des Trägers angepasst werden kann. Sowohl die vorderen Seitenabschnitte 22 als auch die hinteren Seitenabschnitte 20 sind aus einem Vliesstoffmaterial, im dargestellten Fall aus einem PP-Spinnvlies, Pegatex S, Hersteller: Pegas a.s.,Primetickä 86, 66904 Znojmo, CZ, gebildet. Das Flächengewicht des Vliesstoffmaterials der vorderen Seitenabschnitte beträgt 30 g/m². Die Faserstärke der das Vliesstoffmaterial bildenden Fasern beträgt 2 dtex. Die Über-Bauch-Haltekräfte zwischen den Verschlussmitteln 32 und der Außenseite der vorderen Seitenabschnitte 22 betragen vorzugsweise mindestens 58 N/25mm.

Das Flächengewicht des Vliesstoffmaterials der hinteren Seitenabschnitte 20 beträgt im dargestellten Fall 27 g/m².

Die Weiterreißfestigkeiten des Vliesmaterials der hinteren Seitenabschnitte, gemessen in der Längsrichtung 28 beträgt:
Fm: 7,0 N
Fm.sp: 7,2 N
Fsp: 9,8 N.

Die Über-Bauch-Haltekräfte zwischen den Verschlussmitteln 32, 33 und der Außenseite der hinteren Seitenabschnitte 20 sind geringer als die Über-Bauch-Haltekräfte zwischen den Verschlussmitteln 32, 33 und der Außenseite der vorderen Seitenabschnitte 22.

Wie aus Figur 1 erkennbar ist, weisen die hinteren Seitenabschnitte 20 eine größere Flächenerstreckung auf als die vorderen Seitenabschnitte 22.

Die vorderen und hinteren Seitenabschnitte unterscheiden sich damit in zumindest drei ihrer Primäreigenschaften, nämlich dem Flächengewicht, der Verschlusskraft und der Flächenerstreckung.

Der Unterschied in der Verschlusskraft zwischen den vorderen und hinteren Seitenabschnitten veranlasst den Benutzer, die Verschlussmittel 32, 33 bevorzugt an den vorderen Seitenabschnitten 22 festzulegen, was der Passform der Windel förderlich ist. Wie Figur 1 weiter zu erkennen gibt, sind die Beinöffnungsbereiche 50 durch zum Schrittbereich hin kurvenförmig ausgebildete vordere und hintere Seitenabschnitte 20, 22, welche die Seitenabschnittsbeinöffnungsbereiche bilden, sowie durch die sanduhrförmige Konturierung des Hauptteils 4 gebildet. Unter einer sanduhrförmigen Konturierung des Hauptteils wird hier jede Form der Verengung des Hauptteils 4 im Schrittbereich 10 verstanden, also jede, auch jede nicht oder nicht ausschließlich kurvenförmige Form, bei der der Schrittbereich 10 des Hauptteils 4 eine geringere Erstreckung in Querrichtung 30 aufweist als Vorderbereich 6 und/oder Rückenbereich 8 des Hauptteils.

Die Stetigkeit der Beinöffnung 50 ist gebildet durch je, das heißt auf jeder Seite, einen einzigen Schnitt, welcher sowohl die Seitenabschnitte 20, 22 als auch den Hauptteil 4 erfasst und hierbei stetig durch zu trennendes Seitenrand- oder Hauptteilmaterial hindurchgeführt ist. Die Beinöffnung 50 umfasst somit einen vorderen Seitenabschnittsbeinöffnungsbereich 52, einen hinteren Seitenabschnittsbeinöffnungsbereich 51 und den Hauptteilbeinöffnungsbereich 53 (Figur 2).

In der Figur 2 ist außerdem die Positionierung der beinöffnungsnahen Verschlussmittel 32 mit dem Abstand C sowie die Ermittlung der Länge A und der Breite B des unteren Randes 64eines hinteren Seitenabschnitts 20 illustriert. Der hintere Seitenabschnitt 20 ist begrenzt durch einen inneren Rand 60, welcher dem hinteren Seitenrand 41 des Hauptteils entspricht, durch einen äußeren Rand 61, sowie einem oberen Rand 63 und einem unteren Rand 64, welcher die Kontur des Seitenabschnittsbeinöffnungsbereiches bildet. Oberer Rand 63 und unterer Rand 64 verbinden den inneren Rand 60 mit dem äußeren Rand 61. Vorzugsweise verlaufen innerer Rand 60 und äußerer Rand 61 parallel zueinander, weiter bevorzugt verlaufen innerer 60 und/oder äußerer Rand 61 zumindest abschnittsweise parallel zu einer Längsrichtung 28 der Inkontinenzwegwerfwindel. Vorzugsweise weist der innere Rand 60 eine größere Erstreckung D in der Längsrichtung 28 auf als der äußere Rand 61.

Die Länge A ist im Rahmen der vorliegenden Erfindung definiert als die größte Erstreckung des unteren Randes 64 in der Längsrichtung 28. Die Breite B ist definiert als die größte Erstreckung des unteren Randes 64 in der Querrichtung 30. Abstand C ist definiert als die in der Längsrichtung 28 zu ermittelnde kürzeste Distanz zwischen einem beinöffnungsnahen Verschlussmittel 32 und dem unteren Rand 64.

Die weiteren Größen werden im Rahmen der vorliegenden Erfindung wie folgt bestimmt:
Zur Ermittlung des Extrempunktes P ist eine Gerade G durch den inneren Endpunkt H und den äußeren Endpunkt K des unteren Randes 64 gezogen. Mit Bezug auf diese Gerade G ist ein nach außen gewölbter, also konvexer Abschnitt 44 des unteren Randes 64 der hinteren Seitenabschnitte definiert. Im dargestellten Fall weist der untere Rand 64 außerdem einen mit Bezug zu der Geraden G nach innen gewölbten, konkaven Abschnitt 45 auf. Der Extrempunkt P ist jener Punkt des unteren Randes 64 innerhalb des konvexen Abschnittes, welcher einen maximalen Abstand M zwischen der Geraden G und dem unteren Rand 64 aufweist, wobei der Abstand als Lot auf die Gerade G zu messen ist.
Sollte der Extrempunkt P nach dieser Methode nicht eindeutig bestimmbar sein, etwa weil der untere Rand 64 mehr als einen konvexen Abschnitt aufweist oder weil der konvexe Abschnitt mehr als einen Punkt mit maximalem Abstand von der Gerade G aufweist, so wird derjenige Punkt gewählt, welcher in Querrichtung 30 dem inneren Rand 60 am dichtesten liegt.

Die Längserstreckung E des Extrempunktes P ist die in Längsrichtung 28 gemessene Distanz des Extrempunktes P von einer gedachten Linie, welche sich durch den inneren Endpunkt H des hinteren Seitenabschnitts 20 parallel zur Querrichtung 30 erstreckt.

Die Quererstreckung F des Extrempunktes P ist die in Querrichtung 30 gemessene Distanz des Extrempunktes P von einer gedachten Linie, welche sich durch den inneren Endpunkt H des hinteren Seitenabschnitts 20 parallel zur Längsrichtung 28 erstreckt.

In der dargestellten ersten Ausführungsform (Beispiel 1) beträgt das Verhältnis der Länge A des unteren Randes eines hinteren Seitenabschnittes zu der Breite B des unteren Randes eines hinteren Seitenabschnittes 0,63. Das Verhältnis der Längserstreckung E des Extrempunktes P des konvexen Abschnittes zu dessen Quererstreckung F beträgt 0,27.

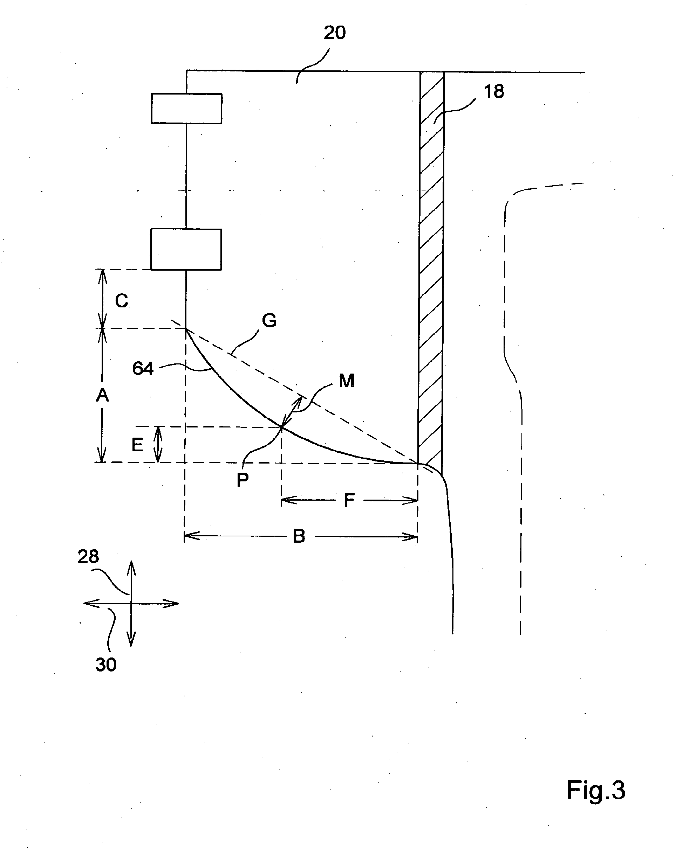

Figur 3 zeigt eine alternative erfindungsgemäße Seitenabschnittgeometrie (Beispiel 2), wobei der untere Rand 64 eines hinteren Seitenabschnittes 20 vollumfänglich in Bezug auf die Gerade G nach außen gewölbt ist, also der untere Rand 64 aus einem einzigen konvexen Abschnitt besteht. Nach Ermittlung des Extrempunkts P als der Punkt, welcher einen maximalen Abstand M von der Geraden G aufweist, lassen sich die weiteren Größen analog wie oben beschrieben ermitteln. Das Verhältnis der Länge A des unteren Randes eines hinteren Seitenabschnittes zu der Breite B des unteren Randes eines hinteren Seitenabschnittes beträgt hier 0,64. Das Verhältnis der Längserstreckung E des Extrempunktes P des konvexen Abschnittes zu dessen Quererstreckung F beträgt 0,40. Der Abstand C des unteren Randes 64 von dem beinöffnungsnahen Verschlussmittel beträgt 6 cm.

Die nachfolgende Tabelle fasst die Größen A-F sowie die Quotienten A/B, E/F, E/A und F/B zusammen:

| | Beispiel 1 | Beispiel 2 |
|---|---|---|
| A in mm | 8,3 | 8,4 |
| B in mm | 13,2 | 13,2 |
| C in mm | 3,0 | 6,0 |
| D in mm | 30,5 | 35,0 |
| E in mm | 1,1 | 2,0 |
| F in mm | 4,1 | 5,0 |
| A/B | 0,63 | 0,64 |
| E/F | 0,27 | 0,40 |
| E/A | 0,13 | 0,24 |
| F/B | 0,31 | 0,38 |

Die Arbeit, gemessen in mNm bis zum Bruch der Materialien betrug bei Beispiel 1 2047 mNm, bei Beispiel 2 2034 mNm und beim einem nicht erfindungsgemäßen Vergleichsbeispiel 1901 mNm. Als Vergleichsbeispiel wurde ein ansonsten baugleicher Inkontinenzartikel geprüft, dessen unterer Rand der hinteren Seitenabschnitte wie in DE102009015041 Figuren 2a, 2b dargestellt einen ausschließlich konkaven Abschnitt aufweist.

Im Rahmen der vorliegenden Erfindung sind und werden die Seitenabschnittausreißfestigkeiten mittels des weiter unten erläuterten Prüfverfahrens gemessen.

Im Rahmen der vorliegenden Erfindung sind und werden die Weiterreißfestigkeiten als Weiterreißkraft nach der in DIN EN ISO 13937-2 spezifizierten Prüfmethode ermittelt. Abweichend davon beträgt die Probenlänge 150 mm. Der mittige Einschnitt weist eine tiefe von 50mm auf. Die Verformungsgeschwindigkeit wird auf 200 mm/min eingestellt. Die Auswertung erfolgt mittels einer elektronischen Einrichtung. Abweichend von DIN EN ISO 13937-2 ist ein auszuwertender Spitzenwert durch einen Kraftanstieg oder einen Kraftabfall von mindestens 0,2 N gekennzeichnet. Es werden somit obere und untere Kraftspitzen bei Ermittlung des Fm.sp berücksichtigt. Neben dem arithmetischen Mittelwert der Kraftspitzen Fm.sp, werden außerdem der Maximalwert aller oberen Kraftspitzen Fsp eines jeweiligen

Prüflings und die über die gesamte auszuwertende Kraftverlaufskurve berechnete mittlere Kraft Fm ermittelt.

Mit der vorliegenden Erfindung ist es somit gelungen, eine Inkontinenzwegwerfwindel mit an den Hauptteil beidseitig angefügten vorderen und hinteren Seitenabschnitten bereitzustellen, welche ein in Gebrauch der Windel weiter verbessertes Seitenabschnittsausreißverhalten aufweist und zugleich den Anlege- und Tragekomfort der Inkontinenzwegwerfwindel ausreichend berücksichtigt.

### Prüfmethode Seitenabschnittsausreißfestigkeit

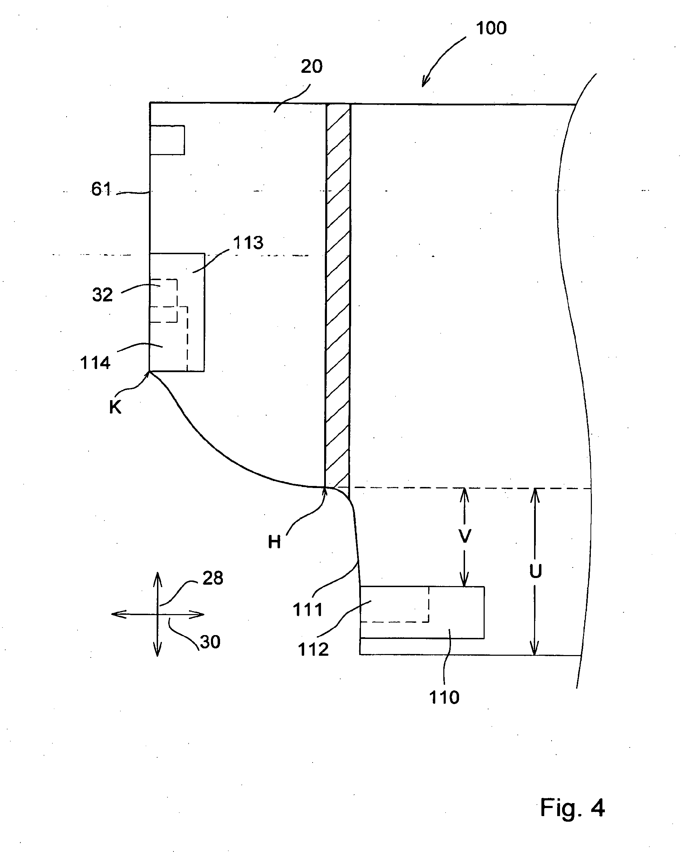

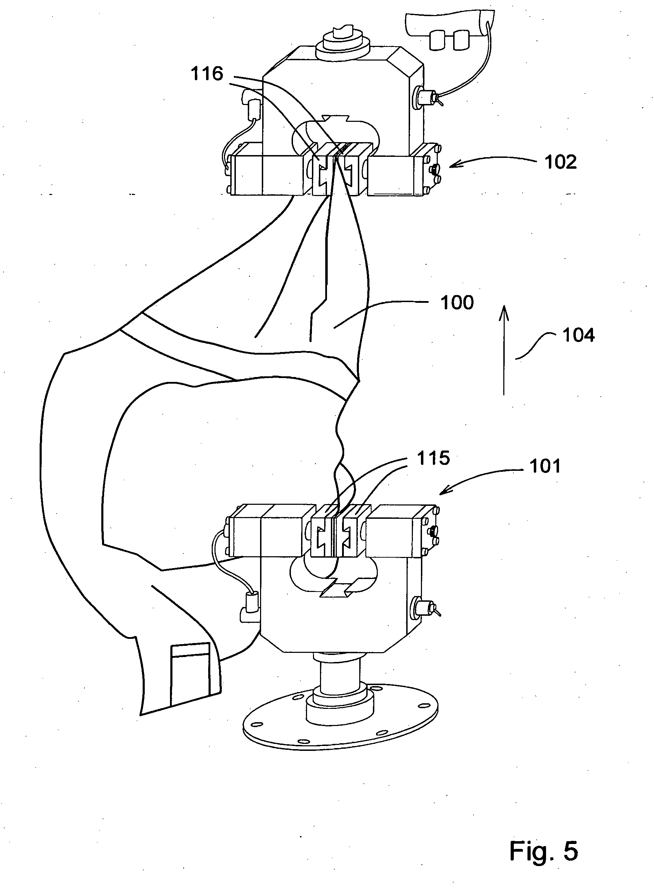

Das Prüfverfahren wird mit Bezug zu Figuren 4 und 5 beschrieben. Die zu prüfenden Inkontinenzartikel werden zunächst über 12 h im Normklima bei 23 °C und 50% relativer Luftfeuchte gelagert.

Eine Inkontinenzwegwerfwindel wird im Abstand U von etwa 150 mm unterhalb des unteren Endpunktes H unter Zerstörung des Hauptteils in Querrichtung 30 durch den gesamten Schrittbereich hindurch getrennt (geschnitten oder gestanzt). An dem so erhaltenen Prüfling 100 wird im Abstand V von 100 mm unterhalb des inneren Endpunktes H zur Materialverstärkung beidseitig, das heißt auf die die Innenseite des Hauptteils und die die Außenseite des Hauptteils bildende Oberfläche ein etwa 50mm breites und etwa 240 mm langes selbstklebendes erstes Textiltape 110 fixiert, das hierzu an dem Längsrand 111 des entsprechenden Schrittbereichsabschnitts umgeschlagen wird, so dass das Tape beidseitig eine Fläche von etwa 50 mm (in Längsrichtung 28) x 120 mm (in Querrichtung 30) bedeckt. Das erste wie auch das weiter unten beschriebene zweite Textiltape dienen lediglich der sichereren Fixierung der entsprechenden Abschnitte in die Klemmen des Zugprüfgerätes wie unten näher beschrieben. Geeignet ist beispielsweise das Omnitape® zu beziehen bei der Paul Hartmann AG, Heidenheim, Deutschland. Bündig mit dem Längsrand 111 und dem oberen Rand des ersten Tapes 110 (das heißt in einem Abstand V von 100 mm unterhalb des hinteren Seitenabschnitts) wird auf dem ersten Tape 110 die erste Position 112 der unteren, feststehenden Klemme 101 des Zugprüfgeräts markiert mit einer Erstreckung in Querrichtung 30 von 60 mm und in Längsrichtung 28 von 30 mm. Unmittelbar oberhalb des äußeren Endpunktes K wird zur Materialverstärkung ebenfalls beidseitig ein etwa 50 mm breites selbstklebendes zweites Textiltape 113 fixiert, so dass das Tape 113 beidseitig eine Fläche von etwa 50 mm (in Querrichtung 30) x 120 mm (in Längsrichtung 28) bedeckt. Ein beinöffnungsnahes Verschlussmittel 32 kann dabei je nach Anordnung des Verschlussmittels von dem zweiten Tape 113 überfangen werden. Bündig mit dem äußeren Rand 61 und dem unteren Rand des zweiten Tapes 113 wird auf dem zweiten Tape 113 die zweite Position 114 der oberen Klemme 102 des Zugprüfgerät markiert mit einer Erstreckung in Querrichtung von 30mm und in Längsrichtung von 60 mm.

Der Prüfling 100 wird sodann in eine Zugprüfmaschine nach DIN EN ISO 75001, Genauigkeitsklasse 1, wie folgt eingespannt:
Die untere, feststehende Klemme 101 wird mittels der beiden Klemmbacken 115 mit einer Breite von 60mm und einer Einspanntiefe von 30 mm an der zuvor markierten ersten Position 112 festgelegt, indem der betreffende Abschnitt zwischen die beiden Klemmbacken 115 der feststehenden Klemme 101 fest eingespannt wird. Die senkrecht über der feststehenden Klemme 101 angeordnete bewegbare Klemme 102 wird mittels der beiden Klemmbacken 116 mit einer Breite von 60mm und einer Einspanntiefe von 30 mm an der zuvor markierten zweiten Position 114 festgelegt, indem der betreffende Abschnitt zwischen die beiden Klemmbacken 116 der bewegbaren Klemme 102 fest eingespannt wird. Durch gesteuerte Bewegungen der bewegbaren Klemme 102 in Richtung des Pfeils 104 wird ein Zugprüfversuch durchgeführt. Die Prüfgeschwindigkeit, mit der die bewegbare Klemme 102 mechanisch senkrecht von der unteren Klemme weg bewegt wird, beträgt 500 mm/min, wobei zunächst eine Vorkraft von 0,5 N (Newton) aufgewendet wird. Insgesamt sollen eine Mindestanzahl von n = 5 Prüfungen durchgeführt werden. Zur Auswertung wird die bis zur Maximalkraft (Bruch der Materialien) aufgewendete Arbeit in [mNm] eines jeden Zugversuches über Integration der Kraft-Dehnungskurve ermittelt und anschließend das arithmetische Mittel der Einzelwerte berechnet.

## Patentansprüche

1. Absorbierende Inkontinenzwegwerfwindel (2) des offenen Typs, mit einem Hauptteil (4), umfassend einen Vorderbereich (6) mit vorderen seitlichen Längsrändern (42), einen Rückenbereich (8) mit hinteren seitlichen Längsrändern (41) und einen in Längsrichtung (28) dazwischen liegenden, zwischen den Beinen eines Benutzers zu liegen kommenden Schrittbereich (10), wobei der Hauptteil (4) einen Saugkörper (12) umfasst, und mit beidseits an den Rückenbereich (8) angefügten hinteren Seitenabschnitten (20) und beidseits an den Vorderbereich (6) angefügten vorderen Seitenabschnitten (22), welche sich in Querrichtung (30) über die seitlichen vorderen und hinteren Längsränder (42, 41) des Hauptteils (4) hinaus erstrecken, und wobei die hinteren Seitenabschnitte (20) erste beinöffnungsnahe Verschlussmittel (32) mit Verschlusshilfen aufweisen und wobei die Verschlussmittel (32) zumindest an der Außenseite der vorderen Seitenabschnitte (22) lösbar festlegbar sind und dadurch der Vorderbereich (6) und der Rückenbereich (8) miteinander verbindbar sind, wobei zur Bildung von hinteren Seitenabschnittsbeinöffnungsbereichen (51) ein unterer Rand (64) der hinteren Seitenabschnitte (20) kurvenförmig ausgebildet ist, wobei der untere Rand (64) der hinteren Seitenabschnitte (20) mit einer Länge A und einer Breite B einen konvexen Abschnitt (44) mit einem Extrempunkt P aufweist, **dadurch gekennzeichnet, dass** das Verhältnis der Länge A zu der Breite B des unteren Randes (64) eines hinteren Seitenabschnittes (20) 0,40-0,90 beträgt und das Verhältnis der Längserstreckung E des Extrempunktes P zu dessen Quererstreckung F 0,15-0,80 beträgt.

2. Absorbierende Inkontinenzwegwerfwindel (2) nach Anspruch 1, **dadurch gekennzeichnet, dass** das Verhältnis der Quererstreckung F des Extrempunktes P zu der Breite B des unteren Randes (64) eines hinteren Seitenabschnittes (20) 0,20-0,60, insbesondere 0,30-0,50 beträgt.

3. Absorbierende Inkontinenzwegwerfwindel (2) nach einem der vorstehenden Ansprüche, **dadurch gekennzeichnet, dass** das Verhältnis der Längserstreckung E des Extrempunktes P zur der Länge A des unteren Randes (64) eines hinteren Seitenabschnittes (20) 0,10-0,40, insbesondere 0,10-0,30 beträgt.

4. Absorbierende Inkontinenzwegwerfwindel (2) nach einem der vorstehenden Ansprüche, **dadurch gekennzeichnet, dass** das Verhältnis der Längserstreckung E eines Extrempunktes P des konvexen Abschnittes (44) zu dessen Quererstreckung F 0, 20-0,60 insbesondere 0,20-0,50 beträgt.

5. Absorbierende Inkontinenzwegwerfwindel (2) nach einem der vorstehenden Ansprüche, **dadurch gekennzeichnet, dass** die Weiterreißfestigkeit Fm des die hinteren Seitenabschnitte (20) bildenden Materials in der Längsrichtung (28) mindestens 4,0 N beträgt.

6. Absorbierende Inkontinenzwegwerfwindel (2) nach einem der vorstehenden Ansprüche, **dadurch gekennzeichnet, dass** das Verhältnis der Länge A des unteren Randes (64) eines hinteren Seitenabschnittes (22) zu der Breite B des unteren Randes (64) eines hinteren Seitenabschnittes (22) 0,50-0,80, insbesondere 0,55-0,75 beträgt.

7. Absorbierende Inkontinenzwegwerfwindel (2) nach einem der vorhergehenden Ansprüche, **dadurch gekennzeichnet, dass** beinöffnungsnahe Verschlussmittel (32) in einem Abstand C von dem unteren Rand der hinteren Seitenabschnitte (20) angeordnet sind, wobei der Abstand C höchsten 8,0 cm, insbesondere höchstens 7,0 cm, weiter insbesondere höchstens 6,5 cm und weiter insbesondere mindestens 0,5 cm beträgt.

8. Absorbierende Inkontinenzwegwefwindel (2) nach einem der vorhergehenden Ansprüche, **dadurch gekennzeichnet, dass** die hinteren Seitenabschnitte mindestens ein weiteres beinöffnungsfernes mit Verschlusshilfen versehenes Verschlussmittel (33) aufweisen.

9. Absorbierende Inkontinenzwegwerfwindel (2) nach einem der vorhergehenden Ansprüche, **dadurch gekennzeichnet, dass** die Weiterreißfestigkeit Fm des die hinteren Seitenabschnitte bildenden Materials in der Längsrichtung (28) mindestens 5,0 N, insbesondere mindestens 6,0 N, insbesondere mindestens 6,5 N und weiter insbesondere höchstens 10,0 N beträgt oder dass die Weiterreißfestigkeit Fm.sp des die hinteren Seitenabschnitte bildenden Materials in der Längsrichtung (28) mindestens 5,5 N, insbesondere mindestens 6,0 N, insbesondere mindestens 6,5 N und weiter insbesondere mindestens 7,0 N und weiter insbesondere höchstens 12 N beträgt oder dass die Weiterreißfestigkeit Fsp des die hinteren Seitenabschnitte bildenden Materials in der Längsrichtung (28) mindestens 5,5 N, insbesondere mindestens 6,0 N, insbesondere mindestens 6,5 N und weiter insbesondere mindestens 7,0 N und weiter insbesondere höchstens 12,0 N beträgt.

10. Absorbierende Inkontinenzwegwerfwindel (2) nach einem der vorhergehenden Ansprüche, **dadurch gekennzeichnet, dass** die Seitenabschnitte (20, 22) aus einem Vliesstoffmaterial gebildet sind oder ein Vliesstoffmaterial umfassen.

11. Absorbierende Inkontinenzwegwerfwindel (2) nach einem der vorstehenden Ansprüche, **dadurch gekennzeichnet, dass** die Erstreckung der Seitenabschnitte (20, 22) über den Seitenrand des Hauptteils (4) hinaus in Querrichtung (30) 10-40 cm, insbesondere 12-35 cm, weiter insbesondere 13-30 cm beträgt.

12. Absorbierende Inkontinenzwegwerfwindel (2) nach einem der vorstehenden Ansprüche, **dadurch gekennzeichnet, dass** die Länge der hinteren Seitenabschnitte (20), also deren Erstreckung in der Längsrichtung (28) mindestens 15 cm, insbesondere mindestens 20 cm, weiter insbesondere mindestens 25 cm und weiter insbesondere mindestens 27 cm und weiter insbesondere höchstens 45 cm beträgt.

## Claims

1. Disposable absorbent incontinence diaper (2) of the open type, having a main part (4), comprising a front region (6) having front lateral longitudinal edges (42), a rear region (8) having rear lateral longitudinal edges (41) and a crotch region (10) that is located in between in the longitudinal direction (28) and comes to lie between the legs of a user, wherein the main part (4) comprises an absorbent pad (12), and having rear side portions (20) that are attached to the rear region (8) on both sides and front side portions (22) that are attached to the front region (6) on both sides, said rear and front side portions extending in the transverse direction (30) beyond the lateral front and rear longitudinal edges (42, 41) of the main part (4), and wherein the rear side portions (20) have first closure means (32) which are close to the leg opening and have closure aids and wherein the closure means (32) can be secured in a detachable manner at least on the outer side of the front side portions (22), as a result of which the front region (6) and the rear region (8) can be connected together, wherein, in order to form rear side-portion leg-opening regions (51), a lower edge (64) of the rear side portions (20) is formed in a curved manner, wherein the lower edge (64) of the rear side portions (20) having a length A and a width B has a convex portion (44) having an extreme point P, **characterized in that** the ratio of length A to width B of the lower edge (64) of a rear side portion (20) is 0.40-0.90 and the ratio of the longitudinal extent E of the extreme point P to the transverse extent F thereof is 0.15-0.80.

2. Disposable absorbent incontinence diaper (2) according to Claim 1, **characterized in that** the ratio of the transverse extent F of the extreme point P to the width B of the lower edge (64) of a rear side portion (20) is 0.20-0.60, particularly 0.30-0.50.

3. Disposable absorbent incontinence diaper (2) according to either of the preceding claims, **characterized in that** the ratio of the longitudinal extent E of the extreme point P to the length A of the lower edge (64) of a rear side portion (20) is 0.10-0.40, particularly 0.10-0.30.

4. Disposable absorbent incontinence diaper (2) according to one of the preceding claims, **characterized in that** the ratio of the longitudinal extent E of an extreme point P of the convex portion (44) to the transverse extent F thereof is 0.20-0.60, particularly 0.20-0.50.

5. Disposable absorbent incontinence diaper (2) according to one of the preceding claims, **characterized in that** the tear propagation resistance Fm of the material forming the rear side portions (20) is at least 4.0 N in the longitudinal direction (28).

6. Disposable absorbent incontinence diaper (2) according to one of the preceding claims, **characterized in that** the ratio of the length A of the lower edge (64) of a rear side portion (22) to the width B of the lower edge (64) of a rear side portion (22) is 0.50-0.80, particularly 0.55-0.75.

7. Disposable absorbent incontinence diaper (2) according to one of the preceding claims, **characterized in that** the closure means (32) close to the leg opening are arranged at a distance C from the lower edge of the rear side portions (20), wherein the distance C is at most 8.0 cm, particularly at most 7.0 cm, more particularly at most 6.5 cm and more particularly at least 0.5 cm.

8. Disposable absorbent incontinence diaper (2) according to one of the preceding claims, **characterized in that** the rear side portions have at least one further closure means (33) which is remote from the leg opening and is provided with closure aids.

9. Disposable absorbent incontinence diaper (2) according to one of the preceding claims, **characterized in that** the tear propagation resistance Fm of the material forming the rear side portions is at least 5.0 N, particularly at least 6.0N, particularly at least 6.5 N and more particularly at least 10.0 N in the longitudinal direction (28), or **in that** the tear propagation resistance Fm.sp of the material forming the rear side portions is at least 5.5 N, particularly at least 6.0 N, particularly at least 6.5 N and more particularly at least 7.0 N and more particularly at most 12 N in the longitudinal direction (28), or in that the tear propagation resistance Fsp of the material forming the rear side portions is at least 5.5 N, particularly at least 6.0 N, particularly at least 6.5 N and more particularly at least 7.0 N and more particularly at most 12.0 N in the longitudinal direction (28).

10. Disposable absorbent incontinence diaper (2) according to one of the preceding claims, **characterized in that** the side portions (20, 22) are formed from a nonwoven material or comprise a nonwoven material.

11. Disposable absorbent incontinence diaper (2) according to one of the preceding claims, **characterized in that** the extent of the side portions (20, 22) beyond the lateral edge of the main part (4) is 10-40 cm, particularly 12-35 cm, more particularly 13-30 cm in the transverse direction (30).

12. Disposable absorbent incontinence diaper (2) according to one of the preceding claims, **characterized in that** the length of the rear side portions (20), that is the extent thereof in the longitudinal direction (28), is at least 15 cm, particularly at least 20 cm, more particularly at least 25 cm and more particularly at least 27 cm and more particularly at most 45 cm.

## Revendications

1. Couche pour incontinence absorbante et jetable (2), du type ouvert, comprenant une partie principale (4), comprenant une région avant (6) avec des bords longitudinaux latéraux avant (42), une région arrière (8) avec des bords longitudinaux latéraux arrière (41) et une région de fourche (10) située entre celles-ci dans la direction longitudinale (28), venant s'appliquer entre les jambes d'un utilisateur, la partie principale (4) comprenant un corps aspirant (12), et comprenant des portions latérales arrière (20) jointes de chaque côté à la région arrière (8) et des portions latérales avant (22) jointes de chaque côté à la région avant (6), qui s'étendent dans la direction transversale (30) au-delà des bords longitudinaux latéraux avant et arrière (42, 41) de la partie principale (4), et les portions latérales arrière (20) présentant des premiers moyens de fermeture (32) proches de l'ouverture des jambes avec des auxiliaires de fermeture et les moyens de fermeture (32) pouvant être fixés de manière détachable au moins au niveau du côté extérieur des portions latérales avant (22), et de ce fait la région avant (6) et la région arrière (8) pouvant être connectées l'une à l'autre, un bord inférieur (64) des portions latérales arrière (20) étant réalisé sous forme courbe pour former des régions d'ouverture des jambes (51) des portions latérales arrière, le bord inférieur (64) des portions latérales arrière (20), avec une longueur A et une largeur B, présentant une portion convexe (44) avec un point d'extrémité P, **caractérisée en ce que** le rapport de la longueur A à la largeur B du bord inférieur (64) d'une portion latérale arrière (20) vaut 0,40-0,90 et le rapport de l'étendue longitudinale E du point d'extrémité P à son étendue transversale F vaut 0,15-0,80.

2. Couche pour incontinence absorbante et jetable (2) selon la revendication 1, **caractérisée en ce que** le rapport de l'étendue transversale F du point d'extrémité P à la largeur B du bord inférieur (64) d'une portion latérale arrière (20) vaut 0,20-0,60, en particulier 0,30-0,50.

3. Couche pour incontinence absorbante et jetable (2) selon l'une quelconque des revendications précédentes, **caractérisée en ce que** le rapport de l'étendue longitudinale E du point d'extrémité P à la longueur A du bord inférieur (64) d'une portion latérale arrière (20) vaut 0,10-0,40, en particulier 0,10-0,30.

4. Couche pour incontinence absorbante et jetable (2) selon l'une quelconque des revendications précédentes, **caractérisée en ce que** le rapport de l'étendue longitudinale E d'un point d'extrémité P de la portion convexe (44) à son étendue transversale F vaut 0,20-0,60, en particulier 0,20-0,50.

5. Couche pour incontinence absorbante et jetable (2) selon l'une quelconque des revendications précédentes, **caractérisée en ce que** la résistance à la déchirure supplémentaire Fm du matériau formant les portions latérales arrière (20) dans la direction longitudinale (28) vaut au moins 4,0 N.

6. Couche pour incontinence absorbante et jetable (2) selon l'une quelconque des revendications précédentes, **caractérisée en ce que** le rapport de la longueur A du bord inférieur (64) d'une portion latérale arrière (22) à la largeur B du bord inférieur (64) d'une portion latérale arrière (22) vaut 0,50-0,80, en particulier 0,55-0,75.

7. Couche pour incontinence absorbante et jetable (2) selon l'une quelconque des revendications précédentes, **caractérisée en ce que** des moyens de fermeture proches de l'ouverture des jambes (32) sont disposés à une distance C du bord inférieur des portions latérales arrière (20), la distance C valant au maximum 8,0 cm, en particulier au maximum 7,0 cm, en outre particulièrement au maximum 6,5 cm et en outre particulièrement au moins 0,5 cm.

8. Couche pour incontinence absorbante et jetable (2) selon l'une quelconque des revendications précédentes, **caractérisée en ce que** les portions latérales arrière présentent au moins un moyen de fermeture (33) supplémentaire éloigné de l'ouverture des jambes et pourvu d'auxiliaires de fermeture.

9. Couche pour incontinence absorbante et jetable (2) selon l'une quelconque des revendications précédentes, **caractérisée en ce que** la résistance à la déchirure supplémentaire Fm du matériau formant les portions latérales arrière dans la direction longitudinale (28) vaut au moins 5,0 N, en particulier au moins 6,0 N, en particulier au moins 6,5 N et en outre en particulier au maximum 10,0 N, ou **en ce que** la résistance à la déchirure supplémentaire Fm.sp du matériau formant les portions latérales arrière dans la direction longitudinale (28) vaut au moins 5,5 N, en particulier au moins 6,0 N, en particulier au moins 6,5 N et en outre en particulier au moins 7,0 N et en outre en particulier au maximum 12 N ou **en ce que** la résistance à la déchirure supplémentaire Fsp du matériau formant les portions latérales arrière dans la direction longitudinale (28) vaut au moins 5,5 N, en particulier au moins 6,0 N, en particulier au moins 6,5 N, et en outre en particulier au moins 7,0 N et en outre en particulier au maximum 12,0 N.

10. Couche pour incontinence absorbante et jetable (2) selon l'une quelconque des revendications précédentes, **caractérisée en ce que** les portions latérales (20, 22) sont formées d'un matériau en étoffe non tissée ou comprennent un matériau en étoffe non tissée.

11. Couche pour incontinence absorbante et jetable (2) selon l'une quelconque des revendications précédentes, **caractérisée en ce que** l'étendue des portions latérales (20, 22) au-delà du bord latéral de la partie principale (4) dans la direction transversale (30) vaut 10-40 cm, en particulier 12-35 cm, en outre en particulier 13-30 cm.

12. Couche pour incontinence absorbante et jetable (2) selon l'une quelconque des revendications précédentes, **caractérisée en ce que** la longueur des portions latérales arrière (20), c'est-à-dire leur étendue dans la direction longitudinale (28), vaut au moins 15 cm, en particulier au moins 20 cm, en outre en particulier au moins 25 cm et en outre en particulier au moins 27 cm et en outre en particulier au maximum 45 cm.
